# EUROPEAN PATENT SPECIFICATION

(11) **EP 1 901 690 B1**
(45) Date of publication and mention of the grant of the patent: **27.08.2014**
(21) Application number: 05759252.9
(22) Date of filing: 14.07.2005
(51) Int. Cl.: A61F 13/49, A61F 13/15, A61F 13/505, A61F 13/496

(54) **A PANT-TYPE ABSORBENT ARTICLE HAVING AN ELASTIC WAISTBAND**
SAUGFÄHIGER ARTIKEL VOM HÖSCHENTYP MIT ELASTISCHEM TAILLENBAND
ARTICLE ABSORBANT DE TYPE CULOTTE AVEC CEINTURE ELASTIQUE

(43) Date of publication of application: 26.03.2008
(73) Proprietor: SCA Hygiene Products AB, 405 03 Göteborg (SE)
(72) Inventor: EDWALL, Kerstin, S-437 35 Lindome (SE)
(74) Representative: Valea AB
(86) International application number: PCT/SE2005/001159
(87) International publication number: WO 2007/008127

(56) References cited:
- EP-A1- 1 192 922
- EP-A2- 1 247 507
- US-A1- 2004 243 086
- US-A1- 2005 107 763

## Description

### Technical field

The present invention refers to a pant-type absorbent article such as a pant diaper, a sanitary pant or incontinence garment, said article having a core region comprising an absorbent core and a chassis, said chassis comprising a front panel, a back panel and an elastic waistband comprising reinforcing elastic elements extending substantially in parallel with a waist edge of the chassis along at least a part of the circumference of said waist edge.

### Background of the invention

Absorbent articles having defined core regions and chassis regions are supposed to have a comfortable fit about the wearer. For pant articles like pant diapers, sanitary pants and incontinence pants it is also desirable that the articles are capable of being pulled up and down over the hips of the wearer to allow the wearer or caregiver to easily put on and remove the article when it has been soiled. It is known to make such absorbent pants with elasticized stretchable side panels and waist portion, usually comprising elastic members, such as elastic threads, contractably affixed between the backsheet and the topsheet.

It is further known to make portions of the chassis of absorbent articles of an elastic material, such as stretch-bonded laminates. Such laminates may include a layer of meltblown elastomeric fibers which have been stretched and sandwiched between outer layers of spunbonded webs.

US 6,552,245 discloses an extensible outer cover for an absorbent article which provides a certain permanent deformation when subjected to a tensile force. The extensible outer cover comprises a necked laminate in the form of one layer of a necked non-elastic film and one layer of an elastic film. The films may be breathable.

WO 03/047488 discloses an elastic laminate comprising an elastic film which on opposite sides is bonded to first and second non-elastic fibrous layers. The laminate is made by bonding the non-elastic fibrous layers to the elastic film layer and subsequently stretching the composite material, causing the non-elastic materials to break. The elastic film material may be of a breathable material. The laminate may be incorporated in an absorbent article.

US 2004/0243086 discloses a disposable pant-like undergarment having stretchable front and back panels, for example made of an elastic laminate. An elastic waistband is secured to the distal edge of at least one of the front and back panels, said elastic waistband having a retracted length which is less than the retracted length of the panel to which is it attached. The elastic waistband comprises a folded non-elastic web member enclosing one or more elongate elastic members.

Further examples of absorbent articles which in part are made of elastic laminates are found in US 6,476,289 and JP 10043235.

International patent applications PCT/SE2004/001004, PCT/SE2004/001005, PCT/SE2004/001415, PCT/SE2005/000309 refer to absorbent articles comprising an outer coversheet in the form of an elastic laminate having improved properties such as cloth-like feel and appearance. An elastic waistband is secured to the waist edge of the outer coversheet.

JP H03-122823 discloses a pant diaper having an elastic waist feature with a number of elastic threads. The waist feature is formed by having a first chassis layer longer at the waist edge than a second chassis layer and folding the protruding part of the first layer over the waist edge of the second layer.

There is however still need for improvement of the properties of absorbent articles comprising an elastic web material, such as an elastic laminate, as an outer coversheet, particularly their fit and appearance at the waist opening. The reinforcing elastic elements in the waistband which provide a tight fit around the waist of the wearer can make the product warm to wear in this area. This could be a problem especially for pant products having a high waist.

### Object and most important features of the invention

An object of the present invention is to provide a pant-type absorbent article of the aforementioned kind which has an improved fit and comfort in the waist area. This has been obtained by the fact that an area of height of at least 5 mm, as seen in the longitudinal direction of the article, adjacent the waist edge of the chassis is free from reinforcing elastic elements along at least a substantial part of the circumference of the waist edge along which the elastic waistband extends, wherein the elastic waistband comprises two or more reinforcing elastic elements extending substantially in parallel at a selected distance from each other, said distance being less than the height of said area free from reinforcing elastic elements adjacent said waist edge.

In one embodiment at least 75% of the circumference of said waist edge of the chassis is free from the reinforcing elastic elements in said area adjacent the terminal edge.

In one aspect of the invention said area free from reinforcing elastic elements adjacent said waist edge has a height of at least 7 mm, preferably at least 10 mm, as seen in the longitudinal direction (y) of the article.

According to one embodiment the elastic waistband comprising said reinforcing elastic elements has one edge attached to a terminal edge of said front and back panels of the chassis and wherein the unattached edge of the waistband is free from reinforcing elastic elements in an area of a length as stated in the preceding claims along at least a substantial part of the circumference of the unattached edge of the waistband.

In a further embodiment the elastic waistband comprising said reinforcing elastic elements is attached to said front and back panels at a selected distance inside their respective terminal waist edge, so that said terminal waist edge protrudes outside the waistband a length as stated in the preceding claims along at least a substantial part of the circumference of the waist edge of the waistband.

In one aspect of the invention the elastic waistband has a retracted length in the transverse direction of the article that is less than the retracted length in the transverse direction of the front and back panels to which is it attached.

In one embodiment at least one of said front and back panels comprises an elastic web material. The elastic web material may be a laminate composed of first and second layers of fibrous material and an elastic film layer located between said first and second fibrous layers. The elastic film layer is preferably breathable.

In one aspect of the invention the elastic laminate has a Water Vapour Transmission Rate according to ASTM E96-00 Procedure D of at least 1500 g/m² 24h, preferably at least 3000 g/m² 24h.

According to one embodiment said elastic laminate comprises first and second fibrous layers of spunbond material, each having a basis weight of between 10 and 35 g/m², preferably between 12 and 30 g/m², more preferably between 10 and 25 g/m², and a breathable elastic film layer having a basis weight between 20 and 80 g/m², preferably between 20 and 60 g/m², said elastic laminate having a Water Vapour Transmission Rate according to ASTM E96-00 Procedure D of at least 1500 g/m² 24h, preferably at least 3000 g/m² 24h.

According to a further embodiment said elastic web material has an elasticity in the transverse direction of the article of at least 30%, preferably at least 50%, more preferably at least 70%, when measured according to the elasticity test specified in the description.

In one aspect of the invention the elastic web material has a basis weight of no more than 100 g/m², preferably no more than 90 g/m².

In a still further embodiment a substantially inelastic web material is arranged in the crotch portion of the article, said inelastic web material being joined to the front and back panels comprising said elastic web material.

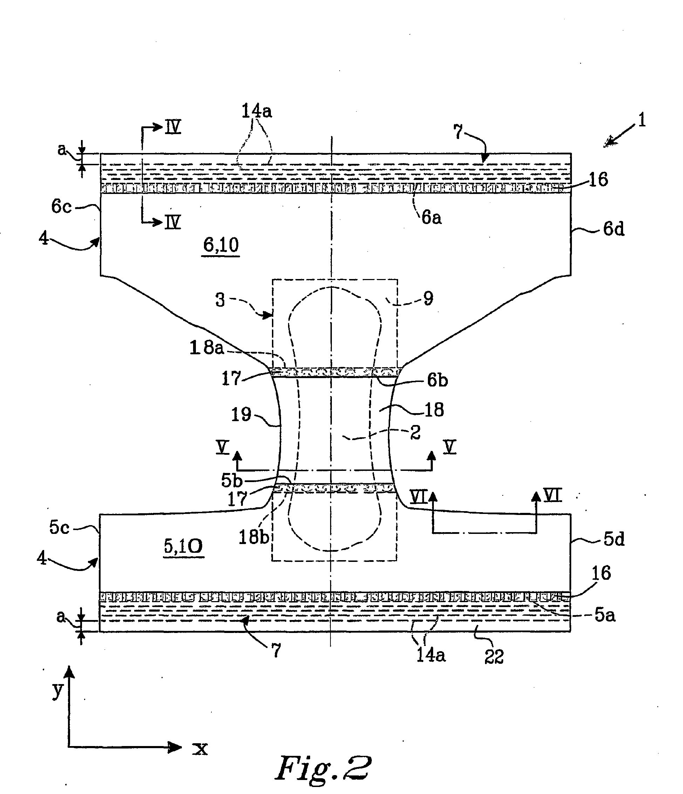

According to one embodiment the surface area of the absorbent core amounts to no more than 30%, preferably not more than 20%, of the total surface area of the article, as measured in a flat state of the article. The term "flat state" herein means in an opened untensioned state, as seen in Figure 2 of the drawings, and in which any tensioned elastic members have been deactivated.

### Brief description of drawings,

The invention will be described in the following in greater detail by way of example and with reference to the accompanying drawings, in which:
Fig. 1 shows a perspective view of a first embodiment of a pant type absorbent article.
Fig. 2 shows is a plan view of the pant article of Fig. 1 in its flat, uncontracted state prior to formation as seen from the garment facing side.
Fig. 3 is a corresponding plan view as in Fig. 2 but showing a second embodiment of a pant article.
Fig. 4 is a cross section according to the line IV-IV in Fig. 2.
Fig. 5 is a cross section according to the line V-V in Fig. 2.
Fig. 6 is a cross section through an elastic laminate according to the line VI-VI in Fig. 2.

### Description of preferred embodiments

The invention will in the following be closer described with reference to some embodiments shown in the accompanying drawings.

### Absorbent article

The term "absorbent article" refers to products that are placed against the skin of the wearer to absorb and contain body exudates, like urine, faeces and menstrual fluid. The invention mainly refers to disposable absorbent articles, which means articles that are not intended to be laundered or otherwise restored or reused as an absorbent article after use. According to the invention pant-type absorbent articles are referred to having a core region and a chassis region surrounding the core region. Examples of such pant-type absorbent articles are pant diapers, sanitary pants and incontinence pants.

The drawings show an embodiment of a pant diaper 1 for an infant or an incontinent adult. Said pant diaper typically comprises an absorbent core 2 located in a core region 3 of the article, and a chassis 4 surrounding the core region. The chassis comprises a front panel 5, a back panel 6 and an elastic waistband 7. The core region 3 is located at least in the crotch portion 19 of the article and extends a certain distance into the front 5 and back panels 6. The crotch portion 19 is herewith defined as the narrow part of the article intended to be worn in the wearer's crotch between the legs.

The article has a longitudinal direction y and a transverse direction x.

The article comprises a liquid permeable topsheet 8 and a liquid impermeable backsheet 9 covering at least the core region 3. The absorbent core 2 is enclosed between the topsheet 8 and the backsheet 9.

### Topsheet

The liquid permeable topsheet 8 can consist of a nonwoven material, e g spunbond, meltblown, carded, hydroentangled, wetlaid etc. Suitable nonwoven materials can be composed of natural fibers, such as wood pulp or cotton fibres, manmade fibres, such as polyester, polyethylene, polypropylene, viscose etc. or from a mixture of natural and manmade fibres. The topsheet material may further be composed of tow fibres, which may be bonded to each other in a bonding pattern, as e.g. disclosed in EP-A-1 035 818. Further examples of topsheet materials are porous foams, apertured plastic films etc. The materials suited as topsheet materials should be soft and non-irritating to the skin and intended to be readily penetrated by body fluid, e.g. urine or menstrual fluid. The topsheet may be different in different parts of the absorbent article.

### Backsheet

The liquid impervious backsheet 9 covering the core region 3 on the garment-facing side of the core is of a liquid impervious material, such as a thin plastic film, e.g, a polyethylene or polypropylene film, a nonwoven material coated with a liquid impervious material, a hydrophobic nonwoven material, which resists liquid penetration or a laminate comprising plastic films and nonwoven materials. The core region backsheet material 9 may be breathable so as to allow vapour to escape from the absorbent core, while still preventing liquids from passing therethrough. Examples of breathable backsheet materials are porous polymeric films, nonwoven laminates from spunbond and meltblown layers, laminates from porous polymeric films and nonwovens. The backsheet 9 is preferably inelastic.

### Elastic web material

The outer coversheet covering the front and back panels 5 and 6 of the chassis 4 comprises an elastic web material 10, which is elastic at least in the transverse x-direction of the article. The elasticity in the x-direction should be at least 30%, preferably at least 50%, more preferably at least 70%, as measured by the elasticity test specified below.

Preferably the elastic web material is elastic also in the γ-direction of the article. However the elasticity in the γ-direction is preferably lower than in the x-direction. The elasticity in the γ-direction should in be at least 20%.

In the embodiment shown and described below the elastic web material is an elastic laminate 10 composed of first and second outer layers of fibrous material 11 and 12 and a middle elastic film layer 13 located between said fibrous layers. However it is understood that other types of elastic web materials may be used, such as elastic nonwoven materials, nonwoven materials which per se are inelastic, but which have been elastified by means of elastic threads etc. The elastic web materials may comprise one layer or two or more layers that have been laminated.

In the elastic laminate shown and described below it is preferred that the outer fibrous layers 11 and 12 are chosen so that they, in combination with the inner elastic film layer 13, give the material high resistance to puncture. They also provide a soft and cloth-like feel to the laminate. Examples of suitable materials are carded webs and spunbond materials. The basis weight of the fibrous material layers should be between 10 and 35 g/m², preferably between 12 and 30 g/m², more preferably between 15 and 25 g/m². Examples of suitable polymers used in the fibrous materials are polyethylene, polyesters, polypropylene and other polyolefin homopolymers and copolymers. Natural fibres, for example cotton, may also be used as long as they provide the required properties. A mixture of polymers can contribute to a higher flexibility of the nonwoven layer, and through this, give the nonwoven material a higher elongation at maximum load. A mixture of polyethylene and polypropylene polymers has proved to provide good results in this respect. A mixture of fibers of different polymers is also possible.

The middle layer 13 is according to one embodiment of the invention an apertured elastic film having a basis weight between 20 and 80 g/m², preferably between 20 and 60 g/m². The film may be of any suitable elastic polymer, natural or synthetic. Some examples of suitable materials for the elastic film are low crystallinity polyethylenes, metallocene-catalyzed low crystallinity polyethylene, ethylene vinyl acetate copolymers (EVA), polyurethane, polyisoprene, butadiene-styrene copolymers, styrene block copolymers, such as styrene/isoprene/styrene (SIS), styrene/butadiene/styrene (SBS), or styrene/ethylene-butadiene/styrene block copolymer. Blends of these polymers may also be used as well as other modifying elastomeric or non-elastomeric materials. One example of a suitable film is an apertured three-layer elastomeric film of PE-SEBS-PE.

The total basis weight of the laminate is preferably 100 g/m² or less, more preferably no more than 90 g/m².

The elastic laminate 10 may be manufactured according to the method disclosed in WO 03/047488, wherein one spunbond layer 11 is applied to the film 13 in a tacky state and will thus bond to the film layer, while the other spunbond layer 12 is adhesively laminated to the film layer 13, using for example a pressure sensitive hot melt adhesive. Alternatively the laminate is manufactured according to a modified version of this known method, wherein the modification involves that the laminate is incrementally stretched (through intermeshing gears, IMG), to a point below the elongation at peak load of at least one of the non-elastic nonwoven layers to retain some strength for at least one of the nonwoven layers. The other layer may also be stretched to a point below its elongation at peak load, or to a point at which it will tear during stretching.

The method disclosed in WO 03/047488 involves stretching of the laminate above the point of failure of the fibrous material, so that the non-elastic layers break completely. Therefore, as described in WO 03/047488, the elongation of the laminate is not limited by the stretch modulus of the non-elastic material.

In a preferred embodiment at least one, preferably both fibrous layers, which are bound to the elastic film, are not, in contrast to the method described in WO 03/047488, completely torn upon manufacture of a laminate according to the present invention. Selection of fibrous materials which have an elongation at maximum load greater than the elasticity of the elastic laminate allows the elastic film to stretch without being hindered by the fibrous layers. Such a selection also ensures that the fibrous layers contribute to the puncture resistance of the laminate, as they are not completely torn or broken during manufacture. Preferably both fibrous layers or at least one of the fibrous layers have an elongation at maximum load that is at least 10% higher than the elasticity of the laminate. This is described in more detail in PCT/SE2004/001005, which is incorporated herein by reference.

The opacity of a material layer is the characteristic ability of the material layer to visually hide from view an underlying object or pattern. The opacity is measured in %, wherein 100% opacity means that nothing can be seen through the material layer and 0% means that the material layer is completely transparent. The opacity is measured by the Opacity Test based on luminous-reflectance-factor data and described in PCT/SE2004/001415.

Opacity of the laminate can be obtained by the incorporation of opacifying fillers into the laminate, particularly into the elastic film. Such pigments can be organic or inorganic dyes, colouring agents, or whitening agents. Inorganic materials such as titanium dioxide, inorganic carbonates, synthetic carbonates, talc, nepheline syenite, magnesium hydroxide, aluminium trihydrate siatomaceous earth, mica, natural or synthetic silicas, calcinated clays and mixtures thereof are preferred examples of opacifying fillers.

The filler is preferably added as a master batch at the extrusion of the film. One example of an appropriate concentration is about 5% filler by weight of the film.

It is further preferred that the elastic laminate 10 has a breathability (Water Vapour Transmission Rate) according to ASTM E96-00 Procedure D of at least 1500 g/m² 24h, preferably at least 3000 g/m² 24h.

The open area of the elastic film layer is preferably at least 8%, more preferably at least 10%. The open area is measured by image analysis methods and is defined as the sum of the hole area divided by the total area of the film sample.

### Absorbent core

The "absorbent core" is the absorbent structure disposed between the two covers of the absorbent article. The absorbent core 2 can be of any conventional kind. Examples of commonly occurring absorbent materials are cellulosic fluff pulp, tissue layers, highly absorbent polymers (so called superabsorbents), absorbent foam materials, absorbent nonwoven materials or the like. It is common to combine cellulosic fluff pulp with superabsorbent polymers in an absorbent core. Superabsorbent polymers are water-swellable, water-insoluble organic or inorganic materials capable of absorbing at least about 20 times its weight and in an aqueous solution containing 0.9 weight percent of sodium chloride. Organic materials suitable for use as a superabsorbent material can include natural materials such as polysaccharides, polypeptides and the like, as well as synthetic materials such as synthetic hydrogel polymers. Such hydrogel polymers include, for example, alkali metal salts of polyacrylic acids, polyacrylamides, polyvinyl alcohol, polyacrylates, polyacrylamides, polyvinyl pyridines, and the like. Other suitable polymers include hydrolyzed acrylonitrile grafted starch, acrylic acid grafted starch, and isobutylene maleic anhydride copolymers and mixtures thereof. The hydrogel polymers are preferably lightly crosslinked to render the material substantially water insoluble. Preferred superabsorbent materials are further surface crosslinked so that the outer surface or shell of the superabsorbent particle, fiber, flake, sphere, etc. possesses a higher crosslink density than the inner portion of the superabsorbent. The superabsorbent materials may be in any form suitable for use in absorbent composites including particles, fibers, flakes, spheres, and the like.

A high absorption capacity is provided by the use of high amounts of super-absorbent material. For an absorbent core comprising a matrix of hydrophilic fibers, such as cellulosic fibers, and superabsorbent material, the proportion of superabsorbent material is preferably between 10 and 90% by weight, more preferably between 30 and 70% by weight.

It is conventional in absorbent articles to have absorbent cores comprising layers of different properties with respect to liquid receiving capacity, liquid distribution capacity and storage capacity. The thin absorbent bodies, which are common in for example baby diapers and incontinence guards, often comprise a compressed mixed or layered structure of cellulosic fluff pulp and superabsorbent polymers. The size and absorbent capacity of the absorbent core may be varied to be suited for different uses such as for infants or for adult incontinent persons.

The absorbent core may further include an acquisition distribution layer placed on top of the primary absorbent body and which is adapted to quickly receive and temporarily store discharged liquid before it is absorbed by the primary absorbent core. Such acquisition distribution layers are well known in the art and may be composed of porous fibrous waddings or foam materials.

### Pant diaper

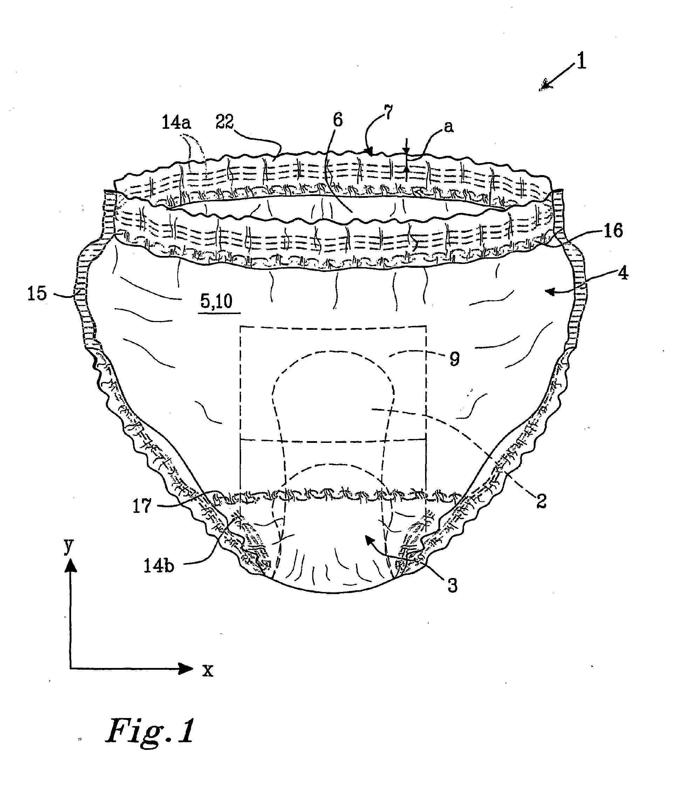

The pant diaper disclosed in Fig. 1 is intended to enclose the lower part of the wearer's trunk like a pair of underwear pants. It comprises a core region 3 located in the narrow crotch portion 19 of the article and extending into the front 5 and back panels 6 of the absorbent pants. A chassis region 4 surrounds the core region 3. The core region 3 is defined as the surface area of the article which is occupied by the absorbent core 2 and the areas outside the core, which are covered by the liquid-impervious backsheet 9. The chassis 4 comprises a front panel 5, a back panel 6 and an elastic waistband 7 secured to the front and back panel. In an alternative embodiment only one of the front 5 and back panels 6 have an elastic waistband 7 secured thereto. Each of the front and back panels 5 and 6 has a waist edge 5a and 6a, a crotch edge 5b and 6b, and a pair of side edges 5c, 6c and 5d and 6d respectively. The front 5 and back panels 6 are joined to each other along their side edges 5c, 6c and 5d, 6d by ultrasonic welds 15, glue strings or the like to form side seams. The elastic waistband portions 7 secured to the front panel 5 and the back panel 6, respectively, are also joined to each other along said side seams. The joined front and back panels 5 and 7 and waistband portions 7 define the waist opening and a pair of leg openings of the pant diaper.

According to one embodiment of the invention the surface area of the absorbent core 2 amounts to no more than 30% of the total surface area of the article, preferably no more than 20%, as measured in a flat state of the article. The term "flat state" herein means in an opened untensioned state, as seen in Figure 2, and in which any tensioned elastic members have been deactivated.

The elastic web material 10 may cover the entire article, including the core region 3 and the entire chassis region 4. However according to a preferred embodiment a substantial part of the crotch portion 19 of the article is free from the elastic web material 10. A "substantial part" used herein refers to at least 50%, preferably at least 75%.

A crotch panel 18, which preferably is a non-elastic material, more preferably a non-elastic nonwoven material, is arranged in the crotch portion of the article and overlaps with the elastic front and back panels 5 and 6. The crotch panel 18 is along its transverse side edges 18a and b joined in an overlapping manner to the front and back panels 5 and 6 respectively by means of ultrasonic welds 17, glue strings or the like.

The elastic waistband 7 comprises a substantially non-elastic nonwoven material that is elasticized by elongate elastic members 14a, such as elastic threads or bands, contractably affixed between material layers, such as nonwoven materials. These elongate elastic members 14a constitute reinforcing elastic elements providing the article with reinforced elasticity in the waist area. Preferably the elastic waistband 7 has a retracted length in the transverse x-direction of the article that which is less than the retracted length in the transverse direction of the front and back panels 5 and 6 to which is it attached, which is illustrated in Fig. 1. In an alternative embodiment however the elastic waistband 7 has a retracted length in the transverse x-direction that which substantially equal to the retracted length in the transverse direction of the front and back panels 5 and 6.

Elastic threads 14b may also be arranged around at least part of the leg openings of the article.

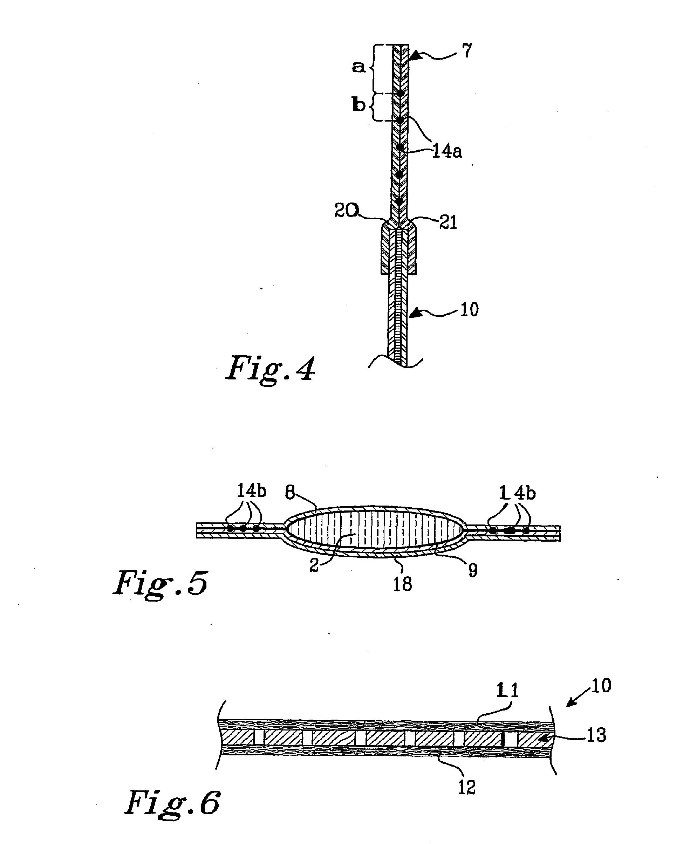

As illustrated in Fig. 5 the elastic waistband 7 may comprise first and second plies 20 and 21 of substantially non-elastic web material enclosing between them at least one elastic element 14a and preferably two or more elastic threads. The substantially non-elastic web material is preferably a nonwoven, The first ply 20 of the waistband is secured to the body-facing side of the elastic web material 10 at the waist edges 5a and 6a of the front and back panels 5 and 6 respectively, and the second ply 21 of the waistband is secured to the opposite, outer side of the elastic web material 10 just opposite the first ply 20.

The first and second plies 20 and 21 are secured to the elastic web material 10 while this is in a stretched condition. The first and second plies 20 and 21 with the elastic web material 10 held there between form a waistband seam 16 joined by ultrasonic welding, glue strings or the like while holding the elastic web material in a stretched condition. This will result in that the first and second plies of non-elastic material 20 and 21 form gathers along the waistband seam 16 when the elastic web material 10 is in a relaxed position.

The elastic waistband 7 may be formed from a double folded substantially non-elastic web material, however may also be formed from two separate plies 20, 21 which are joined together to enclose there between the elastic threads 14.

The waistband seam 16 thus formed provides a very smooth joint between the front and back panels 5, 6 and the waistband 7 both on the wearer facing surface and on the opposite, outer, surface of the article, as only one single ply of web material is joined to each side of the elastic web material 10 in the waistband seam 16, as best illustrated in Fig. 4. The waistband seam 16 will further have a certain degree of elasticity.

In an alternative embodiment the elastic waistband 7 is joined to one of the sides of the elastic web material 10, preferably the side that forms the external side of the pant article. While it is shown that the elastic waistband 7 is joined to both the front and back panels it is also understood that in alternative embodiments only one of the front and back panels 5 and 6 is joined to an elastic waistband 7, wherein the elasticity of the elastic web material 10 is sufficient to keep the other panel in place above the hips of the wearer.

The reinforcing elastic elements 14a exhibit a higher degree of elasticity and higher retractive forces after extension than the elastic web material 10 so as to keep a tight fit around the waist of the wearer. This tight fit may in some cases cause a warm and clammy feeling, especially for pant products having a high waist. In order to increase the comfort of the product in the waist area it has according to the invention been suggested that an area adjacent the waist edge 22 of the article is free from reinforcing elastic elements 14a. This area should have a height, a, as seen in the longitudinal direction y of the article, which is at least 5 mm, preferably at least 7 mm and more preferably at least 10 mm. This will give a small frill around the waist opening which will allow ventilation of this area.

This spacing a between the waist edge 22 and the outermost reinforcing elastic element 14a should be present over at least a substantial part of the circumference of the waist edge 22 along which the elastic waistband 7 extends. A "substantial part" herein means at least 50% of the length, in transverse (y) direction, of the elastic waistband. Preferably at least 75% of said length has said spacing, a, present and more preferably the entire length of the waistband.

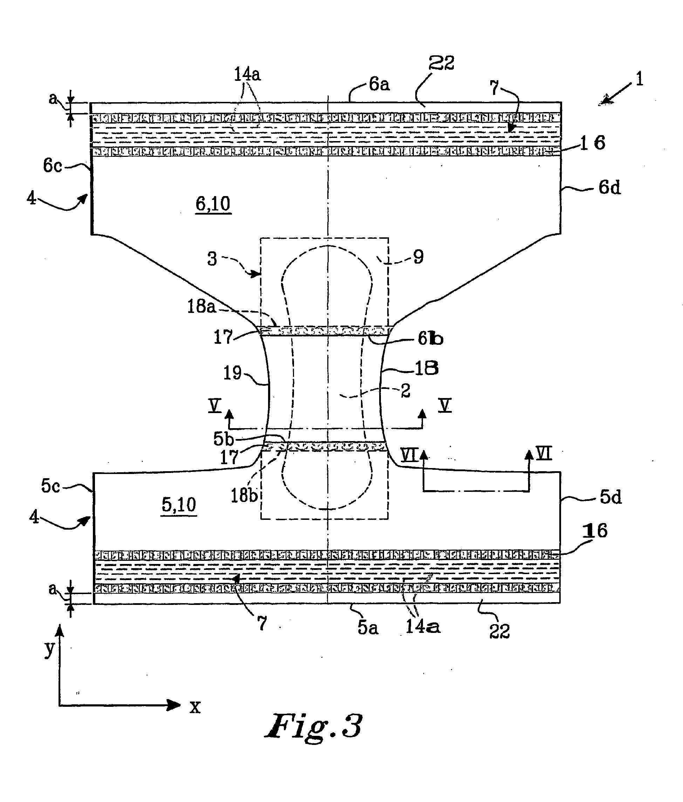

In an alternative embodiment shown in Fig. 3 the elastic waistband 7 is attached to the outside of the elastic web material 10 adjacent but inside the terminal waist edges 5a and 6a of the front and back panels 5 and 6. Thus said terminal edges 5a and 6a protrude outside the waistband 7 a certain distance, said distance corresponding to the spacing, a, described above having a height in γ-direction of at least 5 mm, preferably at least 7 mm and more preferably at least 10 mm. This spacing, a, should be present along at least a substantial part of the length of the waistband 7 in x-direction. The waistband 7 may, as described above, be present along the entire circumference of the waist opening or for example along only one of the front or back panels 5 or 6.

In this embodiment the waist area will have areas of different degrees of elasticity, since the elastic waistband 7 with its reinforcing elastic elements 14a provides a relatively high degree of elasticity and retractive forces, while the area, a, of the front and back panels 5 and 6, protruding outside the elastic waistband 7 provides a relatively lower degree of elasticity and retractive forces. This may result in an improved fit and comfort in the waist area.

The elastic waistband 7 comprises two or more reinforcing elastic elements 14a extending substantially in parallel at a selected distance, b, from each other, said distance b according to one embodiment being less than the height a of said area free from reinforcing elastic elements 14a. When three or more elastic elements 14a are present they may be located at the same or at different distances b from each other.

The liquid-impervious backsheet material 9 underlies the absorbent core 2 and adjacent areas immediately outside the absorbent core 2. The area covered by the liquid-impervious backsheet 9 is defined as the core region 3. The crotch nonwoven material 18 is arranged on the garment-facing side of the liquid-impervious backsheet 9 in the crotch portion of the article. The core region 3 extends into the front and back panels 5 and 6 so that the elastic web material 10 and the liquid impervious backsheet overlap in the outer parts of the core region 3, as seen in Fig. 2 and 3, wherein the elastic web material 10 is arranged on the garment facing side of the liquid impervious backsheet 9.

The elastic web material constitutes the sole component of in parts of the front and back panels 5 and 6 of the chassis 4. In at least 20%, preferably at least 25%, more preferably at least 30% and most preferably at least 40% of the total surface area of the article, as seen in a flat state according to Figure 2 and 3, the elastic web material 10 constitutes the sole component of the chassis.

No additional elasticized side panels joining the front and back panels 5 and 6 are needed when using the elastic web material 10 according to the invention. If desired, additional elasticized side panels may of course be provided, especially in cases where the elastic web material 10 is arranged only in parts of the front and/or back panels.

As stated above the elastic web material 10 has an opacity of at least 40%, preferably at least 50% and more preferably at least 60%. The opacity of the elastic web material provides a cloth-like appearance to the article, which is of particular importance when the article is a pant diaper. Especially in this case, where the elastic web material forms the sole component in considerable surface area regions of the pant diaper, such as large areas of the front and back panels, and the absorbent core covers only relatively small areas, 30% or less, of the article, the appearance of the elastic web material is of great importance for the overall appearance of the article. Thus by making the elastic web material opaque with an opacity of at least 40%, the pant diaper will appear more cloth-like and more like "normal" underwear, than if the elastic web material would have a higher degree of transparency. The opacity is measured by the Opacity Test disclosed in PCT/SE2004/001415.

It is further desired that the elastic web material has a puncture resistance of at least 15N as measured according to ASTM Designation D3763-02. Preferably, the elastic web material of the present invention has a puncture resistance of at least 20N, and more preferably at least 30N.

The elastic web material should preferably have a softness according to Kawabata of at least 20, preferably at least 30 and most preferably at least 40. It is further desired that it has a formability according to Kawabata of no more than 50, preferably no more than 30, more preferably no more than 20 and most preferably no more than 10.It is also desired that the elastic web material has a drapability according to Kawabata of no more than 40. The softness, formability and drapability according to kawabata are measured according to the test methods given in PCT/SE2004/001004.

### Elasticity test

The method measures how an elastic material behaves at repeated load and unload cycles. The sample is stretched to a predetermined elongation and a cyclic movement between 0 and said predetermined elongation is performed. Desired load and unload forces are recorded. The permanent, i.e. remaining, elongation of the relaxed material is measured.

A tensile tester, Lloyd LRX, able to perform cyclic movements and equipped with a printer/plotter or software presentation is used. The sample is prepared by cutting it to a width of 25 mm and a length that is preferably 20 mm longer than the distance between the clamps in the tensile tester.

The tensile tester is calibrated according to the apparatus instructions. The parameters needed for the test (load and unload forces) are adjusted to:
- Crosshead speed: 500 mm/min
- Clamp distance: 50 mm
- Preload: 0.05 N

The sample is placed in the clamps according to the marks and it is made sure that the sample is centered and fastened perpendicularly in the clamps. The tensile tester is started and three cycles between 0 and the predetermined elongation, equal to the highest defined 1^{st} load, are performed. Before the last cycle, the sample is relaxed for 1 minute, then the permanent elongation is measured by stretching the sample until a force of 0.1 N is detected and the elongation is read.

The permanent elongation after relaxation should be less than 10% and is measured by the method above. Thus an elasticity of 30% is defined as that the laminate should have a permanent relaxation after elongation of less than 10% after being exerted to an elongation of 30% in the tensile tester above. An elongation of 30% means an elongation to a length that is 30% longer than the initial length of the sample.

It is understood that although the invention has been described with reference to preferred embodiments, several modifications are possible within the scope of the claims. The invention therefore intends to cover any variations or equivalents which are within known or customary practice within the technical field to which it belongs.

## Claims

1. A pant-type absorbent article (1) such as a pant diaper, a sanitary pant or incontinence pant, said article having a core region (3) comprising an absorbent core (2) and a chassis (4), said chassis comprising a front panel (5), a back panel (6) and an elastic waistband (7) comprising at least one reinforcing elastic element (14a) extending substantially in parallel with a waist edge (22) of the chassis along at least a part of the circumference of said waist edge, said article having a longitudinal (y) and a transverse direction (x),
**characterized in**
**that** an area of height (a) of at least 5 mm, as seen in said longitudinal (y) direction, adjacent said waist edge (22) of the chassis is free from reinforcing elastic elements (14a) along at least a substantial part of the circumference of said waist edge along which the elastic waistband (7) extends, said elastic waistband (7) comprises two or more reinforcing elastic elements (14a) extending substantially in parallel at a selected distance (b) from each other, said distance (b) being less than the height (a) of said area free from reinforcing elastic elements (14a) adjacent said waist edge (22).

2. The absorbent article as claimed in claim 1,
**characterized in**
**that** at least 75% of the circumference of said waist edge (22) of the chassis along which the elastic waistband extend is free from said reinforcing elastic element(s) (14a) in said area adjacent the terminal edge.

3. The absorbent article as claimed in claim 1 or 2,
**characterized in**
**that** said area free from reinforcing elastic element(s) (14a) adjacent said waist edge (22) has a height of at least 7 mm, preferably at least 10 mm, as seen in the longitudinal direction (y) of the article.

4. The absorbent article as claimed in any of the preceding claims,
**characterized in**
**that** the elastic waistband (7) comprising said reinforcing elastic element(s) (14a) has one edge attached to a terminal edge (5a; 6a) of at least one of said front (5) and back panels (6) of the chassis and that the unattached edge of the waistband is free from reinforcing elastic element(s) (14a) in an area of a length as stated in the preceding claims along at least a substantial part of the circumference of unattached edge of the waistband.

5. The absorbent article as claimed in any of the preceding claims,
**characterized in**
**that** the elastic waistband (7) comprising said reinforcing elastic element(s) (14a) is attached to said front (5) and back panels (6) at a selected distance inside their respective terminal waist edge, so that said terminal waist edge protrudes outside the waistband to form an area having a height (a) free from reinforcing elastic elements as stated in the preceding claims along at least a substantial part of the circumference of the waist edge of the waistband.

6. The absorbent article as claimed in any of the preceding claims,
**characterized in**
**that** the elastic waistband (7) has a retracted length in the transverse direction (x) of the article that is less than the retracted length in the transverse direction of the front (5) and back panels (6) to which is it attached.

7. The absorbent article as claimed in any of the preceding claims,
**characterized in**
**that** at least one of said front (5) and back panels (6) comprises an elastic web material (10).

8. The absorbent article as claimed in claim 7,
**characterized in**
**that** the elastic web material is a laminate (10) composed of first and second layers of fibrous material (11,12) and an elastic film layer (13) located between said first and second fibrous layers,

9. The absorbent article as claimed in claim 8,
**characterized in**
**that** said elastic film layer (13) is breathable.

10. The absorbent article as claimed in claim 9,
**characterized in**
**that** said elastic laminate (10) has a Water Vapour Transmission Rate according to ASTM E96-00 Procedure D of at least 1500 g/m² 24h, preferably at least 3000 g/m² 24h.

11. The absorbent article as claimed in any of claims 8-10,
**characterized in**
**that** said elastic laminate (10) comprises first and second fibrous layers (11, 12) of spunbond material, each having a basis weight of between 10 and 35 g/m², preferably between 12 and 30 g/m², more preferably between 15 and 25 g/m² and a breathable elastic film layer (13) having a basis weight between 20 and 80 g/m², preferably between 20 and 60 g/m², said elastic laminate (10) having a Water Vapour Transmission Rate according to ASTM E96-00 Procedure D of at least 1500 g/m² 24h, preferably at least 3000 g/m² 24h.

12. The absorbent article as claimed in any of claims 7-11,
**characterized in**
**that** the elastic web material (10) has a basis weight of no more than 100 g/m², preferably no more than 90g/m².

13. The absorbent article as claimed in any of the claims 7-12,
**characterized in**
**that** the elastic web material (10) constitutes the sole component of the chassis (4) in at least 20%, preferably at least 25%, more preferably at least 30% and most preferably at least 40%, of the total surface area of the article.

14. The absorbent article as claimed in any claims 7-13,
**characterized in**
**that** said elastic web material (10) has an elasticity in the transverse direction (x) of the article of at least 30%, preferably at least 50%, more preferably 70%, when measured according to the elasticity test specified in the description.

15. The absorbent article as claimed in any of claims 7-14,
**characterized in**
**that** the elastic web material (10) has an elasticity in the longitudinal direction (y) of the article of at least 20% when measured according to the elasticity test specified in the description.

16. The absorbent article as claimed in any of the preceding claims,
**characterized in**
**that** a crotch panel (18) of a substantially inelastic web material (180) is arranged in the crotch portion (19) of the article, said crotch panel (18) being joined to the front and back panels (5,6) comprising said elastic web material (10).

17. The absorbent article as claimed in any of the preceding claims,
**characterized in**
**that** the surface area of the absorbent core (2) amounts to no more than 30%, preferably no more than 20%, of the total surface area of the article, as measured in a flat state of the article.

## Patentansprüche

1. Hosenartiger saugfähiger Artikel (1), wie zum Beispiel eine Hosenwindel, eine Damenbinde oder eine Inkontinenzhose, wobei der Artikel einen Kernbereich (3) mit einem saugfähigen Kern (2) und ein Grundelement (4) aufweist, wobei das Grundelement ein vorderes Feld (5), ein hinteres Feld (6) und ein elastisches Taillenband (7) mit mindestens einem verstärkenden elastischen Element (14a) aufweist, das sich im Wesentlichen parallel mit einer Taillenkante (22) des Grundelements entlang zumindest eines Teils des Umfangs der Taillenkante erstreckt und der Artikel eine Längsrichtung (y) und eine Querrichtung (x) aufweist,
**dadurch gekennzeichnet,**
**dass** ein in der Längsrichtung (y) gesehener Höhenbereich (a) von mindestens 5 mm, der an der Taillenkante (22) des Grundelements angrenzt, frei von verstärkenden elastischen Elementen (14a) entlang zumindest eines wesentlichen Teils des Umfangs der Taillenkante ist, entlang der sich das elastische Taillenband (7) erstreckt, wobei das elastische Taillenband (7) zwei oder mehr verstärkende elastische Elemente (14a) aufweist, die sich in einem festgelegten Abstand (b) im Wesentlichen parallel zueinander erstrecken, und der Abstand (b) weniger als die Höhe (a) des Bereichs ist, der frei von an die Taillenkante (22) anzugrenzenden, verstärkenden elastischen Elementen (14a) ist.

2. Saugfähiger Artikel nach Anspruch 1,
**dadurch gekennzeichnet**,
das mindestens 75 % des Umfangs der Taillenkante (22) des Grundelements, entlang dem sich das elastische Taillenband erstreckt, frei von dem elastischen Element(en) (14a) in dem an die Endkante angrenzenden Bereich ist.

3. Saugfähiger Artikel nach Anspruch 1 oder 2,
**dadurch gekennzeichnet,**
**dass** der an die Taillenkante (22) angrenzende Bereich, der frei von einem verstärkenden elastischen Element bzw. Elementen (14a) ist, in der Längsrichtung (y) des Artikels gesehen eine Höhe von mindestens 7 mm, bevorzugt mindestens 10 mm aufweist.

4. Saugfähiger Artikel nach einem der vorstehenden Ansprüche,
**dadurch gekennzeichnet,**
**dass** das elastische Taillenband (7) mit dem verstärkenden elastischen Element(en) (14a) eine Kante aufweist, die an der Endkante (5a; 6a) des vorderen Felds (5) und/oder des hinteren Felds (6) des Grundelements angebracht ist, und dass die nicht angebrachte Kante des Taillenbereichs, wie in den vorstehenden Ansprüchen definiert, in einem Längenbereich frei von dem verstärkenden elastischen Element(en) (14a) ist, und zwar entlang zumindest eines wesentlichen Teils des Umfangs der nicht angebrachten Kante des Taillenbands.

5. Saugfähiger Artikel nach einem der vorstehenden Ansprüche,
**dadurch gekennzeichnet,**
**dass** das elastische Taillenband (7) mit dem verstärkenden elastischen Element(en) (14a) an dem vorderen Feld (5) und dem hinteren Feld (6) mit einem festgelegten Abstand innerhalb ihrer jeweiligen Taillenendkanten angebracht ist, sodass die Taillenendkante zu dem Taillenband nach außen hervorsteht, um einen Bereich mit einer Höhe (a) auszubilden, die, wie in den vorstehenden Ansprüche definiert, frei von verstärkenden elastischen Elementen ist, und zwar entlang zumindest eines wesentlichen Teils des Umfangs der Taillenkante des Taillenbands.

6. Saugfähiger Artikel nach einem der vorstehenden Ansprüche,
**dadurch gekennzeichnet,**
**dass** das elastische Taillenband (7) eine zurückgezogene Länge in der Querrichtung (x) des Artikels aufweist, die kleiner ist als die zurückgezogene Länge in der Querrichtung des vorderen Felds (5) und des hinteren Felds (6) an denen es angebracht ist.

7. Saugfähiger Artikel nach einem der vorstehenden Ansprüche,
**dadurch gekennzeichnet,**
**dass** das vordere Feld (5) und/oder hintere Feld (6) ein elastisches Gewebematerial (10) aufweist.

8. Saugfähiger Artikel nach Anspruch 7,
**dadurch gekennzeichnet,**
**dass** das elastische Gewebematerial ein Laminat (10) ist, das aus einer ersten und zweiten Lage faserigen Materials (11, 12) und einem zwischen der ersten und zweiten faserigen Lage angeordneten elastischen Folienlage (13) zusammengesetzt ist.

9. Saugfähiger Artikel nach Anspruch 8,
**dadurch gekennzeichnet,**
**dass** die elastische Folienlage (13) atmungsaktiv ist.

10. Saugfähiger Artikel nach Anspruch 9,
**dadurch gekennzeichnet,**
**dass** das elastische Laminat (10) eine Wasserdampfdurchlässigkeit nach ASTM E96-00 Prozedur D von mindestens 1500 g/m²24h, bevorzugt von mindestens 3000 g/ m²24h aufweist.

11. Saugfähiger Artikel nach einem der Ansprüche 8 bis 10,
**dadurch gekennzeichnet,**
**dass** das elastische Laminat (10) eine erste und zweite fasrige Lage (11, 12) aus Spinnvliesmaterial aufweist, wobei jede ein Flächengewicht zwischen 10 und 35 g/m², bevorzugt zwischen 12 und 30 g/m², am bevorzugtesten zwischen 15 und 25 g/m² aufweist und eine atmungsaktive elastische Folienlage (13) mit einem Flächengewicht zwischen 20 und 80 g/m², bevorzugt zwischen 20 und 60 g/m², wobei das elastische Laminat (10) eine Wasserdampfdurchlässigkeit nach ASTM E96-00 Prozedur D von mindestens 1500 g/m²24h, bevorzugt von mindestens 3000 g/m²24h aufweist.

12. Saugfähiger Artikel nach einem der Ansprüche 7 bis 11,
**dadurch gekennzeichnet,**
**dass** das elastische Gewebematerial (10) ein Flächengewicht von nicht mehr als 100 g/m², bevorzugt von nicht mehr als 90 g/m² aufweist.

13. Saugfähiger Artikel nach einem der Ansprüche 7 bis 12,
**dadurch gekennzeichnet,**
**dass** das elastische Gewebematerial (10) die einzige Komponente des Grundelements (4) in zumindest 20 %, bevorzugt mindestens 25 %, am bevorzugtesten mindestens 30 % und am meisten bevorzugt in mindestens 40 % des gesamten Flächenbereichs des Artikels ausbildet.

14. Saugfähiger Artikel nach einem der Ansprüche 7 bis 13,
**dadurch gekennzeichnet,**
**dass** das elastische Gewebematerial (10) eine gemäß des in der Beschreibung gemessenen Elastizitätstests gemessene Elastizität in der Querrichtung (x) des Artikels von mindestens 30 %, bevorzugt von mindestens 50 % und am bevorzugtesten von mindestens 70 % aufweist.

15. Saugfähiger Artikel nach einem der Ansprüche 7 bis 14,
**dadurch gekennzeichnet,**
**dass** das elastische Gewebematerial (10) eine gemäß des in der Beschreibung spezifizierten Elastizitätstests gemessene Elastizität in der Längsrichtung (y) des Artikels von mindestens 20 % aufweist.

16. Saugfähiger Artikel nach einem der vorstehenden Ansprüche,
**dadurch gekennzeichnet,**
**dass** ein Schrittfeld (18) aus einem im Wesentlichen unelastischen Gewebematerial (180) in dem Schrittbereich (19) des Artikels angeordnet ist, wobei das Schrittfeld (18) mit dem vorderen und hinteren Feld (5, 6), die das elastische Gewebematerial (10) aufweisen, verbunden ist.

17. Saugfähiger Artikel nach einem der vorstehenden Ansprüche,
**dadurch gekennzeichnet,**
**dass** der Flächenbereich des saugfähigen Kerns (2) nicht mehr als 30 %, bevorzugt nicht mehr als 20 % der in einem flachen Zustand des Artikels gemessenen Gesamtfläche des Artikels ausmacht.

## Revendications

1. Article absorbant (1) du type culotte tel qu'une couche-culotte, une culotte hygiénique ou une culotte contre l'incontinence, ledit article comportant une zone noyau (3) comprenant un noyau absorbant (2) et un support (4), ledit support comprenant un panneau avant (5), un panneau arrière (6) et une bande de taille élastique (7) comportant au moins un élément élastique de renforcement (14a) qui s'étend substantiellement parallèlement à un bord de taille (22) du support, le long d'au moins une partie de la circonférence dudit bord de taille, ledit article ayant une direction longitudinale (y) et une direction transversale (x),
**caractérisé en ce qu'**
une zone de hauteur (a) d'au moins 5 mm, vu dans ladite direction longitudinale (y) adjacente audit bord de taille (22) du support est exempte d'éléments élastiques de renforcement (14a) le long d'au moins une partie substantielle de la circonférence dudit bord de taille le long duquel s'étend la bande de taille élastique (7), **en ce que** ladite bande de taille élastique (7) comporte deux ou plusieurs éléments élastiques de renforcement (14a) qui s'étendent substantiellement parallèlement à une distance choisie (b) les uns des autres, ladite distance (6) étant inférieure à la hauteur (a) de ladite surface exempte d'éléments élastiques de renforcement (14a) à proximité dudit bord de taille (22).

2. Article absorbant selon la revendication 1,
**caractérisé en ce qu'**
au moins 75 % de la circonférence dudit bord de taille (22) du support le long duquel s'étend la bande de taille élastique sont exempts desdits éléments élastiques de renforcement (14a), dans la zone proche du bord terminal

3. Article absorbant selon la revendication 1 ou 2
**caractérisé en ce que**
ladite zone exempte d'éléments élastiques de renforcement (14a) à proximité dudit bord de taille (22) a une hauteur d'au moins 7 mm, de préférence d'au moins 10 mm, comme on le voit dans la direction longitudinale (y) de l'article.

4. Article absorbant selon l'une quelconque des revendications qui précèdent,
**caractérisé en ce que**
la bande élastique de taille (7) comprenant ledit/lesdits élément(s) élastique(s) de renforcement (14a) présente un bord qui est fixé à un bord terminal (5a, 6a) d'au moins un desdits panneaux avant (5) et arrière (6) du support et **en ce que** le bord non fixé de la bande de taille est exempt d'éléments élastiques de renforcement (14a) sur une zone d'une longueur telle qu'exposée dans les revendications qui précèdent le long d'au moins une partie substantielle de la circonférence du bord non fixé de la bande de ceinture.

5. Article absorbant selon l'une quelconque des revendications qui précèdent,
**caractérisé en ce que**
la bande élastique de taille (7) comportant lesdits éléments élastiques de renforcement (14a) est fixée auxdits panneaux avant (5) et arrière (6) à une distance choisie à l'intérieur de leur bord de taille terminal respectif, de telle manière que ledit bord de taille terminal fait saillie à l'extérieur de la bande de ceinture en vue de former une zone ayant une hauteur (a) exempte d'éléments élastiques de renforcement comme exposé dans les revendications qui précèdent le long d'au moins une partie substantielle de la circonférence du bord de taille de la bande de taille.

6. Article absorbant selon l'une quelconque des revendications qui précèdent,
**caractérisé en ce que**
la bande de taille élastique (7) a une longueur à l'état rétracté dans la direction transversale (x) de l'article qui est inférieure à la longueur à l'état rétracté dans la direction transversale des panneaux avant (5) et arrière (6) auxquels elle est fixée.

7. Article absorbant selon l'une quelconque des revendications qui précèdent,
**caractérisé en ce qu'**
au moins un des panneaux avant (5) et arrière (6) comprend un matériau en bande élastique (10).

8. Article absorbant selon la revendication 7
**caractérisé en ce que**
le matériau en bande élastique est un stratifié (10) constitué d'une première et d'une deuxième couches de matériau fibreux (11, 12) et d'une couche de film élastique (13) située entre lesdites première et deuxième couches fibreuses.

9. Article absorbant selon la revendication 8
**caractérisé en ce que**
ladite couche de film élastique (13) est perspirante.

10. Article absorbant selon la revendication 9
**caractérisé en ce que**
ledit stratifié élastique (10) a un taux de transmission de vapeur d'eau selon la norme ASTM E96-00 Procedure D d'au moins 1500 g/m² par 24 heures, de préférence d'au moins 3000 g/m² par 24 heures.

11. Article absorbant selon l'une quelconque des revendications 8 à 10,
**caractérisé en ce que**
ledit stratifié élastique (10) comprend des première et deuxième couches fibreuses (11, 12) de matériau lié au filage, dont chacune a une masse surfacique comprise entre 10 et 35 g/m², de préférence comprise entre 12 et 30 g/m², plus préférablement comprise entre 15 et 25 g/m², et une couche de film élastique perspirante (13) qui a une masse surfacique comprise entre 20 et 80 g/m², de préférence comprise entre 20 et 60 g/m², ledit stratifié élastique (10) ayant un taux de transmission de vapeur d'eau selon la norme ASTM E96-00 Procedure D d'au moins 1500 g/m² par 24 heures, de préférence d'au moins 3000 g/m² par 24 heures.

12. Article absorbant selon l'une quelconque des revendications 7 a 11,
**caractérisé en ce que**
le matériau en bande élastique (10) a une masse surfacique qui n'est pas supérieure à 100 g/m², de préférence qui n'est pas supérieure à 90 g/m².

13. Article absorbant selon l'une quelconque des revendications 7 a 12,
**caractérisé en ce que**
le matériau en bande élastique (10) constitue l'unique composant du support (4) dans au moins 20 %, de préférence au moins 25 %, plus préférablement au moins 30 %, le plus préférablement au moins 40 % de la surface totale de l'article.

14. Article absorbant selon l'une quelconque des revendications 7 a 13,
**caractérisé en ce que**
ledit matériau en bande élastique (10) a une élasticité dans la direction transversale (x) de l'article d'au moins 30 %, de préférence d'au moins 50 %, plus préférablement de 70 %, mesurée selon l'essai d'élasticité exposé dans la description.

15. Article absorbant selon l'une quelconque des revendications 7 a 14,
**caractérisé en ce que**
le matériau en bande élastique (10) a une élasticité dans la direction longitudinale (y) de l'article d'au moins 20 %, mesurée selon l'essai d'élasticité exposé dans la description.

16. Article absorbant selon l'une quelconque des revendications qui précèdent,
**caractérisé en ce qu'**
un panneau d'entrejambe (16) constitué d'un matériau en bande substantiellement non élastique (180) est agencé dans la partie entrejambe (19) de l'article, ledit panneau d'entrejambe (18) étant relié aux panneaux avant et arrière (5, 6) qui comprennent ledit matériau en bande élastique (10).

17. Article absorbant selon l'une quelconque des revendications qui précèdent,
**caractérisé en ce que**
la surface du noyau absorbant (2) ne s'élève pas à plus de 30 %, de préférence pas à plus de 20 % de la surface totale de l'article mesurée, l'article étant à l'état stable.
